(19) Europäisches Patentamt / European Patent Office / Office européen des brevets

(11) **EP 3 581 931 B1**

(12) **EUROPEAN PATENT SPECIFICATION**

(45) Date of publication and mention
of the grant of the patent:
**23.12.2020 Bulletin 2020/52**

(51) Int Cl.:
***G01N 33/543*** *(2006.01)*  ***G01N 33/558*** *(2006.01)*
***G01N 21/78*** *(2006.01)*  ***G01N 33/483*** *(2006.01)*

(21) Application number: **18752048.1**

(22) Date of filing: **23.01.2018**

(86) International application number:
**PCT/JP2018/002027**

(87) International publication number:
**WO 2018/147077 (16.08.2018 Gazette 2018/33)**

(54) **IMMUNOLOGICAL TESTING DEVICE**

IMMUNOLOGISCHE TESTVORRICHTUNG

DISPOSITIF D'ESSAI IMMUNOLOGIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.02.2017 JP 2017021611**

(43) Date of publication of application:
**18.12.2019 Bulletin 2019/51**

(73) Proprietor: **Fujifilm Corporation
Minato-ku
Tokyo 106-8620 (JP)**

(72) Inventor: **TAKEUCHI, Yuta
Ashigarakami-gun
Kanagawa 258-8538 (JP)**

(74) Representative: **Klunker IP
Patentanwälte PartG mbB
Destouchesstraße 68
80796 München (DE)**

(56) References cited:
**EP-A1- 2 320 232        EP-A2- 1 111 386
EP-A2- 2 703 803        WO-A1-2012/086376
WO-A2-2013/014540    CN-A- 105 353 115
JP-A- 2009 115 521      JP-A- 2010 101 715
JP-A- 2013 140 182      JP-A- 2014 035 290
JP-A- 2016 212 024      US-A1- 2009 253 213
US-A1- 2009 253 213**

**Description**

**BACKGROUND OF THE INVENTION**

1. Field of the Invention

The present invention relates to an immunological examination apparatus.

2. Description of the Related Art

Immunological examination apparatuses that detect or quantify antigens, such as viruses in a sample, are used for examination of viral infectious diseases, such as influenza, utilizing an antigen-antibody reaction. In this type of immunological examination apparatus, typically, a sample solution is dropped onto a chromatographic test piece. The dropped sample solution flows through the test piece in a longitudinal direction, and passes through a reaction section provided in the test piece. The reaction section is colored by catching analysis object substance in the sample solution by an antigen-antibody reaction, the reaction section is imaged, and antigens are detected or quantified on the basis of the concentration of the reaction section in the obtained image.

In a case where a longitudinal axis of the test piece is inclined with respect to the horizontal, the flow of the sample solution flowing through the test piece in the longitudinal direction becomes faster or slower due to the influence of gravity that acts on the sample solution, and the amount of the sample solution passing through the reaction section within a predetermined time changes. For this reason, even in a case where the concentration of the antigen in the sample solution is the same, a difference is caused in the coloration intensity of the reaction section, and analysis accuracy decreases. Thus, in an examination apparatus described in JP2009-115521A, the inclination of a longitudinal axis of a test piece with respect to the horizontal is detected, and in a case where the inclination exceeds a predetermined range, a warning showing an abnormality in posture of the test piece is output, and the analysis is stopped.

US2009/0253213 discloses an automated apparatus for reading immunochromatographic test strips comprising an optoelectronic scanning system, an inclination sensor for monitoring the horizontal alignment of the apparatus and an analysis unit. The inclination sensor detects the inclination oft the whole apparatus in the longitudinal direction and in the transverse direction and the shut-off/interruption signal is generated when a certain inclination is exceeded.

EP 2 320 232 discloses another automated apparatus for reading immunochromatographic test strips comprising an imaging unit, an acceleration sensor and a processing unit. Alignment of the test strip with an optical system is ensured by connecting both elements with a holder.

JP 2014/035290 discloses a portable fluorophotometer including an inclination sensor for monitoring the inclination of a cuvette (inside the housing) with respect to the horizontal. The signal of this sensor is processed by a control circuit for generating a warning signal/error message when the inclination fo the cuvette exceeds a certain level.

CN 105 353 115 discloses an automated apparatus for reading immunochromatographic test strips comprising a photodetection system, a sloping platform, a translation platform and a data acquisition & conversion system. The test strip is placed on the sloping platform and an analysis unit provides for correct alignment of the strip with the optical path by controlling the inclination of the slope unit.

EP 2 703 803 discloses an apparatus for reading immunochromatographic test strips comprising a camera-based imaging unit, an analysis unit and two position sensors which monitor proper insertion of the test strip and provide the corresponding signals for triggering the capture of images of the control/test regions.

WO2013/014540 discloses an automated apparatus for reading immunochromatographic test strips comprising means which enables the strip to be optically interrogated along the longitudinal axis.

EP 1 111 386 discloses a test strip provided with means (a marking with a IR dye) for monitoring ensuring alignment with the optical system once inserted in a spectrophotometer.

**SUMMARY OF THE INVENTION**

In the examination apparatus described in JP2009-115521A, the inclination of a lateral axis of the test piece is not taken into consideration. In a case where the lateral axis of the test piece is inclined with respect to the horizontal, a sample solution may be biased toward an end part, which is disposed relatively below, out of both end parts of the test piece in a lateral direction, and unevenness in the lateral direction may occur in the coloration of a reaction section.

There is a case where the concentration of the reaction section as a base for the analysis is, for example, an average value of concentration distribution in the lateral direction from a viewpoint of reducing the influence of noise resulting from dust adhering to the test piece. In a case where the unevenness in the lateral direction has occurred in the coloration of the reaction section, the concentration of the reaction section may be different from an original concentration due to the averaging, and thus, the analysis accuracy decreases. In this way, there is concern that the analysis accuracy may decrease even depending on the inclination of the test piece in the lateral direction.

The invention has been made in view of the above circumstances and an object thereof is to improve analysis accuracy irrespective of the posture of a chromatographic test piece.

An immunological examination apparatus of an aspect of the invention comprises an imaging unit that acquires an image of a chromatographic test piece on which a sample solution is made to flow in a longitudinal direction; an inclination detecting unit that detects an inclination of a lateral axis of the chromatographic test piece with respect to the horizontal; and an analysis unit that sets a single analysis region to the image acquired by the imaging unit, changes a range of the analysis region in a lateral direction on the basis of the inclination of the lateral axis detected by the inclination detecting unit, and analyzes the sample solution on the basis of concentration information of the analysis region that is changed.

According to the invention, analysis accuracy can be improved irrespective of the posture of the chromatographic test piece.

## BRIEF DESCRIPTION OF THE DRAWINGS

Fig. 1 is a perspective view of an example of a cartridge that houses a chromatographic test piece for describing an embodiment of the invention.
Fig. 2 is a schematic view of the chromatographic test piece of Fig. 1.
Fig. 3 is a block view of an example of an immunological examination apparatus for describing an embodiment of the invention.
Fig. 4 is a schematic view of an imaging unit of the immunological examination apparatus of Fig. 3.
Fig. 5 is a schematic view illustrating an example of the chromatographic test piece in which a reaction section is colored.
Fig. 6 is a graph illustrating concentration information created from an image of the chromatographic test piece of Fig. 5.
Fig. 7 is a schematic view illustrating another example of the chromatographic test piece in which the reaction section is colored.
Fig. 8 is a schematic view illustrating an example of setting of an analysis region for the image of the chromatographic test piece.
Fig. 9 is a schematic view illustrating an example of setting of an analysis region for the image of the chromatographic test piece.
Fig. 10 is a schematic view illustrating an example of setting of an analysis region for the image of the chromatographic test piece.
Fig. 11 is a flowchart illustrating the processing to be executed by a control unit of the immunological examination apparatus of Fig. 3.
Fig. 12 is a schematic view illustrating an example of setting of an analysis region for the image of the chromatographic test piece.
Fig. 13 is a schematic view illustrating an example of setting of an analysis region for the image of the chromatographic test piece.
Fig. 14 is a schematic view illustrating an example of setting of an analysis region for the image of the chromatographic test piece.
Fig. 15 is a flowchart illustrating the processing to be executed by the control unit of the immunological examination apparatus of Fig. 3.

## DESCRIPTION OF THE PREFERRED EMBODIMENTS

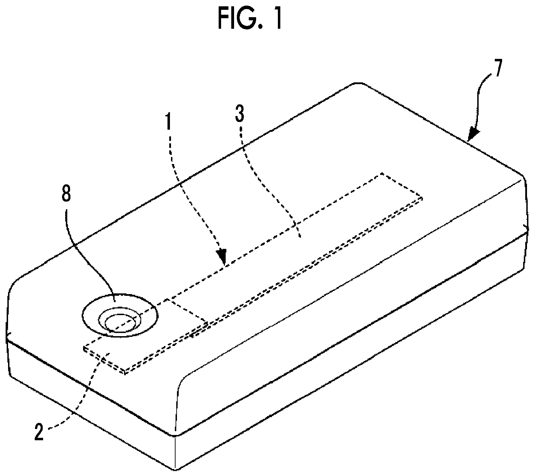

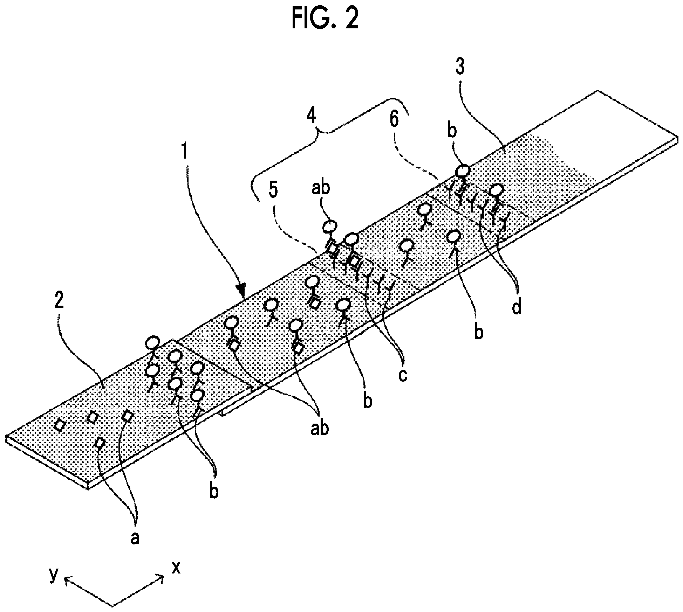

Figs. 1 and 2 illustrate an example of a chromatographic test piece to be used for analysis of a sample solution and a cartridge housing the chromatographic test piece, which are provided for describing an embodiment of the invention.

A chromatographic test piece (hereinafter referred to as a test piece) 1 illustrated in Figs. 1 and 2 is used for analysis in which antigens, such as viruses in the sample solution, are detected or quantified utilizing an antigen-antibody reaction. The test piece 1 is, for example, a beltlike thin piece made of porous materials, such as cellulose, and is typically white. The test piece 1 comprises a dropping section 2 provided at an end part on one side in a longitudinal direction x, and a spreading section 3 provided adjacent to the dropping section 2 in the longitudinal direction x. The sample solution is dropped onto the dropping section 2, the sample solution dropped onto the dropping section 2 flows from the dropping section 2 to the spreading section 3 by the capillary phenomenon, and further flows through the spreading section 3 in the longitudinal direction x toward an end part opposite to the dropping section 2.

The dropping section 2 is provided with labeled antibodies b labeled with golden colloidal particles. The labeled antibodies b are dissolved in the sample solution dropped onto the dropping section 2, and are bonded with antigens a to form antigen-antibody complexes ab in a case where the antigen a is included in the sample solution. The antigen-antibody complexes ab are moved riding on the flow of sample solution, and the surplus labeled antibodies b, which have remained without being bonded with the antigens a, are moved riding on the flow of the sample solution.

The spreading section 3 is provided with a reaction section 4. The reaction section 4 has a determination line 5 for detecting or quantifying the antigens a in the sample solution, and has and further has a control line 6 for detecting that the sample solution has appropriately flowed to the test piece 1 in the present example. The determination line 5 and the control line 6 are provided across the spreading section 3 in a lateral direction y orthogonal to the longitudinal direction x, and are the control line 6 is provided downstream of the flow of the sample solution than the determination line 5.

First capture antibodies c to be bonded with the antigens a are fixedly provided on the determination line 5 for detecting or quantifying the antigens a in the sample solution. The antigen-antibody complexes ab, which are moved riding on the flow of the sample solution, are captured by the first capture antibodies c of the determination line 5, and are fixed to the determination line 5. The determination line 5 is colored with the golden colloidal particles adhering to the labeled antibodies b of the antigen-antibody complexes ab by the antigen-antibody complexes ab being fixed to the determination line 5, and the coloration of the determination line 5 becomes stronger as the antigen-antibody complexes ab to be fixed become increase. The coloration of the determination line 5 is optically detected as changes in absorbance, and the antigens a in the sample solution are detected or quantified.

Second capture antibodies d to be bonded with the labeled antibodies b are fixedly provided in the control line 6 for detecting that the sample solution has appropriately flowed to the test piece 1. The surplus labeled antibodies b moved riding on the flow of the sample solution pass by the determination line 5 without being captured by the first capture antibodies c, are captured by the second capture antibodies d of the control line 6, and are fixed to the control line 6. The control line 6 is colored with the golden colloidal particles adhering to the labeled antibodies b as the labeled antibodies b being fixed to the control line 6, and the coloration of the control line 6 becomes stronger as the labeled antibodies b to be fixed increases. That is, the control line 6 is colored by coming into contact with the sample solution irrespective of whether the antigens a are included in the sample solution. The coloration of the control line 6 is optically detected as changes in absorbance, and is detected that the sample solution has appropriately flowed to the test piece 1.

The test piece 1 is housed in a cartridge 7 and used. The cartridge 7 is provided with an opening 8 that faces the dropping section 2 of the housed test piece 1, and the sample solution is dropped onto the dropping section 2 through the opening 8. The cartridge 7 is made of transparent resin materials, and the coloration of the reaction section 4 (the determination line 5 and the control line 6) of the test piece 1 is optically detected through the cartridge 7.

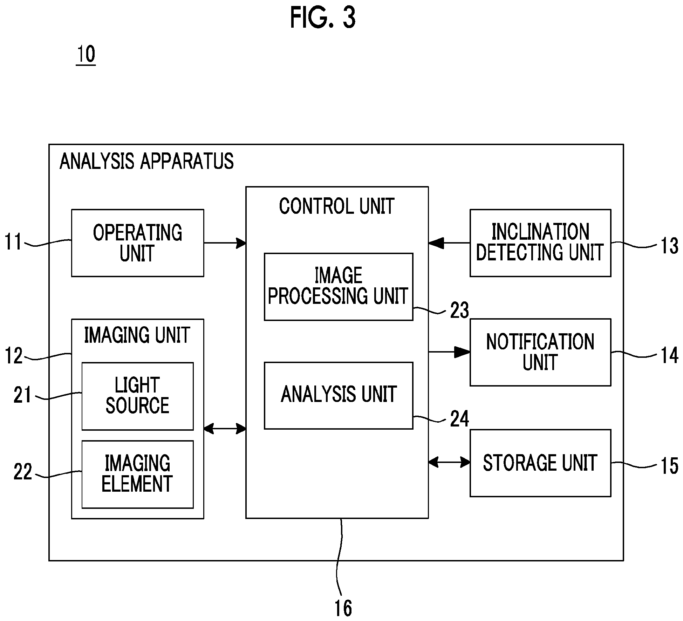

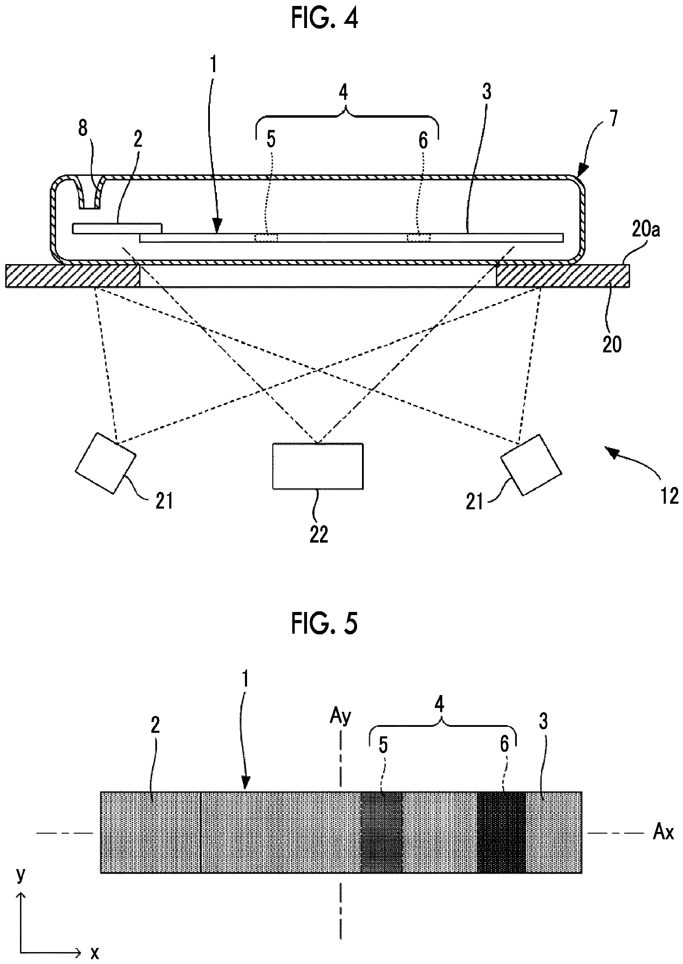

Figs. 3 and 4 illustrate an example of the immunological examination apparatus that analyzes using the test piece 1.

The immunological examination apparatus 10 comprises an operating unit 11, an imaging unit 12, an inclination detecting unit 13, a notification unit 14, a storage unit 15, and a control unit 16 that controls the operation of the operating unit 11, the imaging unit 12, the inclination detecting unit 13, the notification unit 14, and the storage unit 15.

The operating unit 11 receives various instructions (for example, an analysis start instruction and the like) of an operator. The operating unit 11 is constituted of, for example, hardware keys, such as switches. The instructions received by the operating unit 11 are input to the control unit 16.

The imaging unit 12 optically detects the coloration of the reaction section 4 (the determination line 5 and the control line 6) of the test piece 1. The imaging unit 12 includes an installation unit 20 in which the cartridge 7 housing the test piece 1 is installed, a light source 21, such as a light emitting diode (LED), and an imaging element 22, such as a charge-coupled device (CCD) or a complementary metal oxide semiconductor (CMOS). The test piece 1 of the cartridge 7 installed in the installation unit 20 is illuminated by the light source 21, and is imaged by the imaging element 22 in the illuminated state. The acquired image of the test piece 1 is input to the control unit 16.

The inclination detecting unit 13 detects the inclination of an upper surface 20a of the installation unit 20, on which the cartridge 7 is installed, with respect to the horizontal. As the inclination of the upper surface 20a of the installation unit 20 with the horizontal is detected, the inclination of an axis Ay (hereinafter referred to as a lateral axis), extending in the lateral direction y of the test piece 1 installed in the installation unit 20, with respect to the horizontal, and the inclination of an axis Ax (hereinafter referred to as a longitudinal axis), extending in the longitudinal direction x, with respect to the horizontal are indirectly detected. Although not particularly limited, the inclination detecting unit 13 is constituted by sensors, such as a mechanical sensor in which a load cell and a weight are combined with each other, an acceleration sensor, a gyro sensor, and a magnetic sensor. The detected inclination is input to the control unit 16.

The notification unit 14 notifies the operator of various kinds of information (for example, analysis results and the like). The notification unit 14 may include, for example, display panels, such as a liquid crystal display (LCD) and an organic electro-luminescence display (OELD), and may give a notification of the information by displaying images and texts on display screens of the display panels. Additionally, the notification unit 14 may include indicating lamps, such as a light emitting diode (LED), and may give a notification of the information by lighting, blinking, and the like of the indicating lamps. Additionally, the notification unit 14 may include buzzers, and may give a notification of the information by voice.

The storage unit 15 stores control programs and control data to be executed by the control unit 16, and stores various kinds of information, such as the analysis results. The storage unit 15 is constituted of, for example, storage media, such as a flash memory, a hard disk, a read-only memory (ROM), and a random access memory (RAM).

The control unit 16 controls the operation of the operating unit 11, the imaging unit 12, the inclination detecting unit 13, the notification unit 14, and the storage unit 15 by operating in accordance with the control programs. Additionally, the control unit 16 also functions as an image processing unit 23 and an analysis unit 24 by operating in accordance with the control programs.

The image processing unit 23 creates concentration information obtained by digitizing the coloration intensity of the reaction section 4 (the determination line 5 and the control line 6) appearing on the image of the test piece 1 depending on the concentration (luminance and/or brightness) of the image.

The analysis unit 24 detects or quantifies the antigens a in the sample solution on the basis of the coloration intensity of the determination line 5 indicated depending on the concentration information, and detects that the sample solution has appropriately flowed to the test piece 1 on the basis of the coloration intensity of the control line 6 indicated depending on the concentration information. Then, the analysis unit 24 creates the above analysis results.

The hardware structure of the control unit 16 that performs various kinds of processing as the image processing unit 23 and the analysis unit 24 include exclusive electric circuits, which are processors having circuit configurations exclusively designed to execute specific processing, such as a central processing unit (CPU) that is a general-purpose processor, a programmable logic device (PLD) that is a processor capable of changing a circuit configuration after manufacture of a field programmable gate array (FPGA) or the like, and an application specific integrated circuit (ASIC).

The respective processing units (the image processing unit 23, the analysis unit 24, and the like) of the control unit 16 may be constituted of one of above various processors for each processing unit, or may be constituted of a combination of two more same or different types of processors (for example, a combination of a plurality of the FPGAs or a combination of the CPU and the FPGA). Additionally, the plurality of processing units may be constituted of one processor.

As an example in which the plurality of processing units are constituted of the one processor, firstly, as represented by a computer, such as a client or a server, there is a form in which one processor is constituted of a combination of one or more CPUs and software and this processor functions as the plurality of processing units. Secondly, as represented by a system-on-chip (SOC) or the like, there is a form in which a processor, which realizes functions of an overall system including the plurality of processing units with one integrated circuit (IC) chip, is used. In this way, the various processing units are configured by using one or more of the above various processors as the hardware structure(s). Moreover, the hardware structures of these various processors are more specifically circuitries in which circuit elements, such as semiconductor elements, are combined together.

In the above configuration, the control unit 16 executes a predetermined analysis process in a case where the analysis start instruction is input from the operating unit 11 by operating in accordance with the control programs. The predetermined analysis process includes a step of counting lapse of a predetermined reaction time from the start of analysis, a step of making the imaging unit 12 image the test piece 1 after the lapse of the predetermined reaction time, a step of creating the concentration information obtained by digitizing the coloration intensity of the reaction section 4 (the determination line 5 and the control line 6) appearing on the acquired image of the test piece 1 depending on the concentration of the image, and a step of creating analysis results on the basis of the created concentration information. In addition, the reaction time is the time during which the reaction section 4 is in contact with the sample solution, and the predetermined reaction time is the time during which the reaction section 4 is colored with sufficient intensity for detection in a case where the sample solution including antigens in a predetermined concentration has flowed through the horizontally-placed test piece 1, and is appropriately set.

Then, the control unit 16 notifies the notification unit 14 of the analysis results created through the above predetermined analysis process, and stores the analysis results in the storage unit 15.

Fig. 5 illustrates an example of the test piece 1 in which the reaction section 4 is colored.

The example of Fig. 5 shows a case the sample solution flows through the test piece 1 in a state where the test piece 1 is placed horizontally, and the above predetermined reaction time has lapsed. The sample solution had flowed through the test piece 1 in the longitudinal direction x, that is, a leading end of a flow in the sample solution has arrived at the end part of the test piece 1 opposite to the dropping section 2, and the reaction section 4 (the determination line 5 and the control line 6) is colored.

Then, since the test piece 1 is horizontal, the sample solution flows through the test piece 1 substantially equally over the entire length in the lateral direction y without being biased toward one end of the test piece 1 in the lateral direction y. The determination line 5 and the control line 6 that cross the test piece 1 in the lateral direction y are substantially homogeneously colored over the entire length in the lateral direction y.

The test piece 1 is white in a state where the sample solution is not flowing. However, as the sample solution flows and the test piece 1 is wetted, the color tone of the region of the test piece 1, which has been wetted with the sample solution, becomes dark compared to white, and the color tone of the colored reaction section 4 (the determination line 5 and the control line 6) becomes still darker than the region of the test piece 1 that has been wetted in the sample solution.

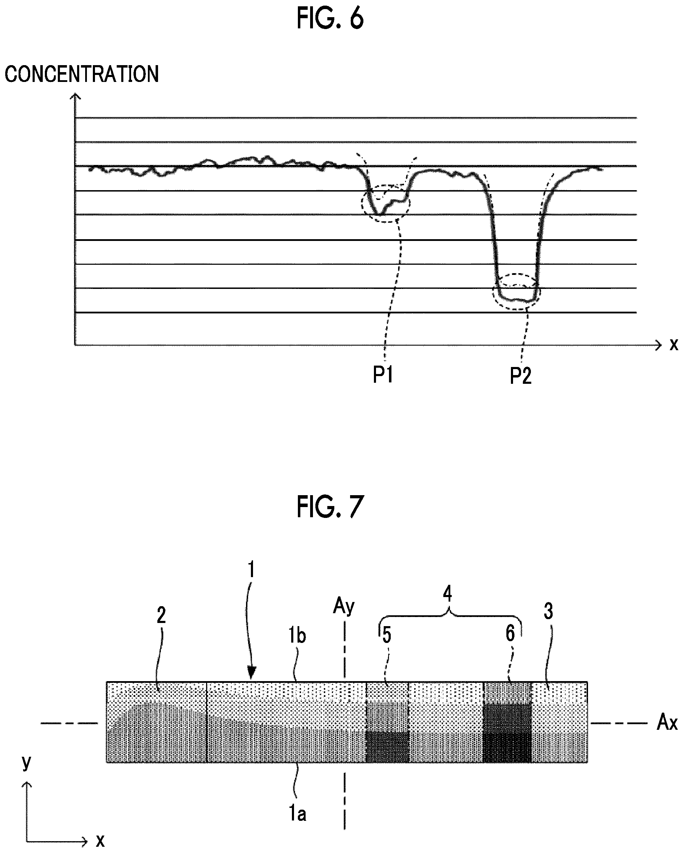

Fig. 6 illustrates an example of concentration information created from the image of the test piece 1 of Fig. 5.

The concentration information is obtained by digitizing the image of the test piece 1 depending on the concentration (luminance and/or brightness), and shows concentration changes in the longitudinal direction x of the test piece 1.

The technique for the digitization is not particularly limited. However, for example, In a case where the digitization is performed depending on the luminance assuming that image signals in RGB (G: green component, R: red component, and B: blue component) format are output from the imaging unit 12, concentrations D at respective pixel positions can be calculated according to Expression (1) by using an R value, a G value, a B value of respective pixels that constitutes the image, a coefficient X for the R value, a coefficient Y for the G value, and a coefficient Z for the B value.

$$D = X \times R + Y \times G + Z \times B \cdots (1)$$

In addition, although X, Y, and Z of Expression (1) can be set to, for example, X = 0.299, Y = 0.587, and Z = 0.144, these values are not limited to this. In the above example, all of the R value, the G value, and the B value are reflected on the luminance. However, for example, Y = 0 and Z = 0 may be set, and only the R value may be reflected on the luminance, or Z = 0 may be set and only the R value and the G value may be reflected on the luminance. X, Y, and Z can be appropriately set depending on, for example, the sensitivities of the respective color components R, G, and B with respect to the coloration of the reaction section 4 (the determination line 5 and the control line 6).

Additionally, in a case where the digitization is performed depending on the luminance, the concentrations D at the respective pixel positions can be calculated by the following Expression (2) by using a maximum value Max and a minimum value Min of the R value, the G value, and the B value of the respective pixels.

$$D = (Max + Min)/2 \cdots (2)$$

Additionally, the concentrations D can also be the maximum value Max of the R value, the G value, and the B value of the respective pixels.

In addition, in digitizing an image depending on the concentrations, white is made to have the highest concentration value. In the above digitization depending on the luminance and the above digitization depending on the luminance, (R, G, B) = (0, 0, 0) may be made black and (R, G, B) = (1, 1, 1) may be made white, assuming that the ranges of R, G, and B are 0 and 1.

Then, an average value of the concentrations at the respective pixel positions is calculated for every y-direction pixel row that extends in the lateral direction y of the test piece 1 from a viewpoint of reducing influence of noise resulting from dust adhering to the test piece 1, the cartridge 7, or the like. Concentration information showing concentration changes in the longitudinal direction x of the test piece 1 is created by the average value in each y-direction pixel row.

In the concentration information created in this way, the colored determination line 5 appears as a peak P1, and the colored control line 6 appears as a peak P2. The analysis unit 24 detects or quantifies the antigens a in the sample solution on the basis of on a concentration value of the peak PI, and detects that the sample solution has appropriately flowed to the test piece 1 on the basis of a concentration value of the peak P2.

Fig. 7 illustrates another example of the test piece 1 in which the reaction section 4 is colored.

The example of Fig. 7 shows a case where the lateral axis Ay of the test piece 1 is inclined with respect to the horizontal, and the sample solution flows through the test piece 1 in a state where an end part 1a out of the end part la and an end part 1b in the lateral direction y of the test piece 1 is disposed relatively below, and the above predetermined reaction time has lapsed. In addition, the end part 1a out of the end part 1a and the end part 1b in the lateral direction y of the test piece 1 being disposed relatively below means the position of the end part 1a in the vertical direction is lower than the position of the end part 1b in the vertical direction. The sample solution is biased toward the end part 1a and flows, and unevenness in which the coloration intensity becomes relatively strong on the end part 1a side and the coloration intensity becomes relatively weak on the end part 1b side occurs in the coloration of the reaction section 4 (the determination line 5 and the control line 6).

In this case, in a case where concentration information is created and in a case where the average value of the concentrations at the respective pixel positions is calculated over the entire length in the lateral direction y for every y-direction pixel row that extends in the lateral direction y of the test piece 1, for example, a concentration value of a pixel group on the end part 1b side having a relatively low coloration intensity is included in the concentration value of the peak P1 corresponding to the determination line 5. According to the above digitization depending on the luminance and/or the brightness, the concentration value of the pixel group on the end part 1b side with the relatively low coloration intensity (a relatively bright color tone) becomes relatively high. Thus, the concentration value of the peak P1 becomes higher than an original concentration value obtained in a case where the sample solution flows through the test piece 1 in a state where the test piece 1 is horizontally placed as illustrated by a one-dot chain line in Fig. 6. Similarly, the concentration value of the peak P2 corresponding to the control line 6 also becomes higher than the original concentration value.

In this way, in a case where the concentration values of the peak P1 and the peak P2 are different from the original concentration values due to the inclination of the lateral axis Ay of the test piece 1 with respect to the horizontal, the accuracy of the analysis based on the concentration values of the peak P1 and the peak P2 decreases. Thus, in the method of creating the concentration information to be described below, it is assumed that a single analysis region is set in the image of the test piece 1 and analysis is performed on the basis of concentration information of this analysis region, and the range of an analysis region in the lateral direction is changed on the basis of the inclination of the lateral axis Ay of the test piece 1 with respect to the horizontal.

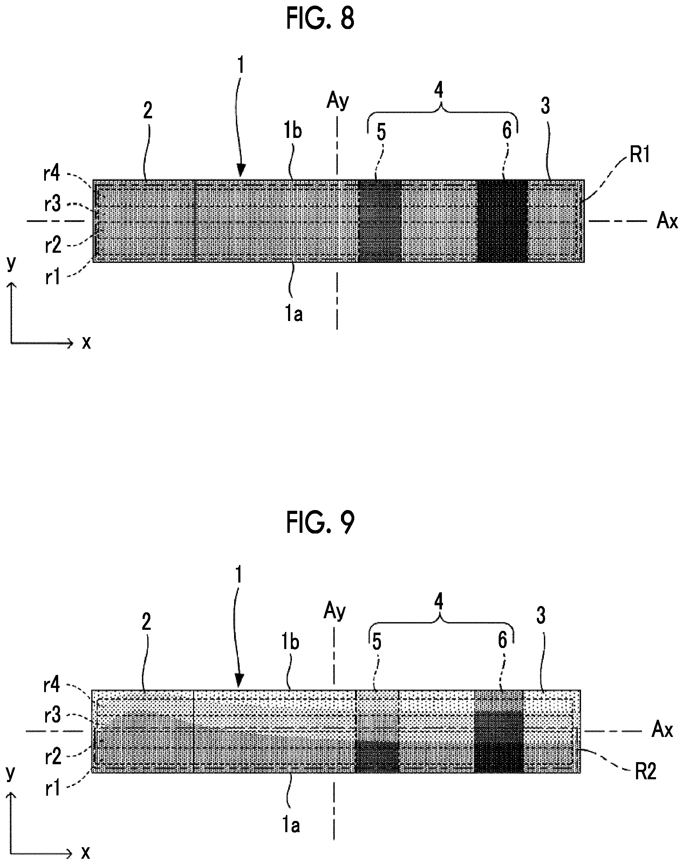

Figs. 8 to 10 illustrate examples of setting of the analysis region according to the inclination of the lateral axis Ay of the test piece 1 with respect to the horizontal.

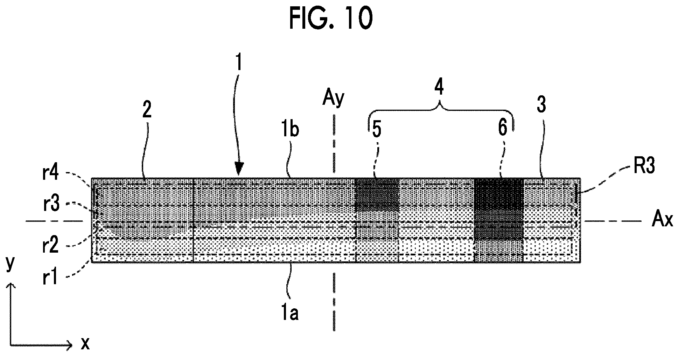

The example of Fig. 8 shows an analysis region set in a case where the lateral axis Ay of the test piece 1 is horizontal, the example of Fig. 9 shows an analysis region set in a case where the lateral axis Ay of the test piece 1 is inclined with respect to the horizontal and the end part 1a out of the end part 1a and the end part 1b of the test piece 1 in the lateral direction y is disposed relatively below, and the example of Fig. 10 shows an analysis region set in a case where the lateral axis Ay of the test piece 1 is inclined with respect to the horizontal and the end part 1b out of the end part 1a and the end part 1b in the lateral direction y of the test piece 1 is disposed relatively below.

First, the image of the test piece 1 is divided into a plurality of pieces in the lateral direction y. In addition, in the illustrated example, the image is divided into four in the lateral direction y. However, the image may be divided into two or may be divided into five or more.

As illustrated in Fig. 8, in a case where the lateral axis Ay of the test piece 1 is horizontal, the analysis region R1 is set to a range that covers four divided region r1 to divided region r4, that is, to the entire image of the test piece 1.

Meanwhile, as illustrated in Fig. 9, in a case where the lateral axis Ay of the test piece 1 is inclined with respect to the horizontal and the end part 1a of the test piece 1 in the lateral direction y is disposed relatively below, an analysis region R2 is set to a range that covers two adjacent divided regions r1 and divided region r2 on the end part 1a side. That is, the range of the analysis region R2 in the lateral direction y is reduced toward the end part 1a side, which is disposed relatively below, out of the end part 1a and the end part 1b of the test piece 1 in the lateral direction y, with respect to the analysis region R1 illustrated in Fig. 8.

In a case where the concentration information is created, the average value of the concentrations at the respective pixel positions is calculated within the analysis region R2 for every y-direction pixel row that extends in the lateral direction y of the test piece 1. Accordingly, the concentration value of the pixel group on the end part 1b with the relatively low coloration intensity is not included in the concentration value of the peak P1 corresponding to the determination line 5, and a difference between the concentration value of the peak P1 and the original concentration value becomes small. Similarly, a difference between the concentration value of the peak P2 corresponding to a control line 6 and the original concentration value also becomes small.

Additionally, as illustrated in Fig. 10, in a case where the lateral axis Ay of the test piece 1 is inclined with respect to the horizontal and the end part 1b of the test piece 1 in the lateral direction y is disposed relatively below, an analysis region R3 is set to a range that covers two adjacent divided region r3 and divided region r4 on the end part 1b side. That is, the range of the analysis region R3 in the lateral direction y is reduced toward the end part 1b side, which is disposed relatively below, out of the end part 1a and the end part 1b of the test piece 1 in the lateral direction y, with respect to the analysis region R1 illustrated in Fig. 8.

In a case where the concentration information is created, an average value of concentrations at respective pixel positions is calculated within the analysis region R3 for every y-direction pixel row that extends in the lateral direction y of the test piece 1, and a difference between the concentration value of the peak P1 corresponding to the determination line 5 and the original concentration value becomes small, and a difference between the concentration value of the peak P2 corresponding to the control line 6 and the original concentration value also becomes small.

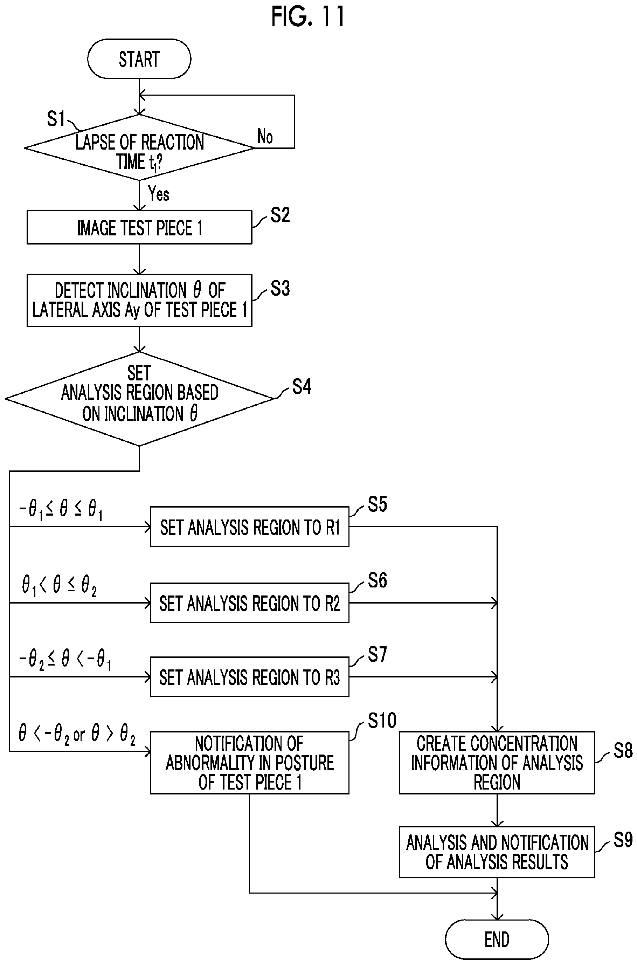

Fig. 11 illustrates a flow of the processing, in which the range of the analysis region is changed in accordance with the inclination of the lateral axis Ay of the test piece 1 with respect to the horizontal, to be executed the control unit 16.

In a case where an analysis start instruction is input, the control unit 16 counts the lapse of a predetermined reaction time $t_1$ from the start of the analysis (Step S1). The predetermined reaction time $t_1$ is the time during which the reaction section 4 is colored with sufficient intensity for detection in a case where the sample solution including antigens in a predetermined concentration has flowed to the horizontally-placed test piece 1, and can be set to, for example, 10 minutes.

The control unit 16 makes the imaging unit 12 image the test piece 1 after the lapse of the predetermined reaction time (Step S2). An image of the test piece 1 acquired by the imaging unit 12 is input to the control unit 16 (image processing unit 23).

Then, the control unit 16 acquires the inclination of the installation unit 20 of the imaging unit 12 with respect to the horizontal from the inclination detecting unit 13, and detects an inclination θ of the lateral axis Ay of the test piece 1 installed in the installation unit 20 with respect to the horizontal (Step S3).

The control unit 16 sets an analysis region on the basis of the inclination θ of the lateral axis Ay of the test piece 1 detected in Step S3 with respect to the image of the test piece 1 acquired in Step S2 (Step S4). In the present example, the control unit 16 applies two threshold values $\theta_1$ and threshold value $\theta_2$ to the inclination θ, and sets the analysis region. In addition, the threshold value $\theta_1$ and the threshold value $\theta_2$ are all positive values, and satisfy $\theta_1 < \theta_2$. Additionally, an inclination direction in which the end part la out of the end part 1a and the end part 1b of the test piece 1 in the lateral direction y is disposed relatively below is set as a normal direction.

In a case where the inclination $\theta$ satisfies $-\theta_1 \leq \theta \leq \theta_1$, the control unit 16 determines that no bias of the sample solution has occurred. Although not particularly limited, the threshold value $\theta_1$ can be, for example, 10°. Then, the control unit 16 set the analysis region to the analysis region R1 illustrated in Fig. 8, that is, the entire image of the test piece 1 (Step S5).

In a case where the inclination $\theta$ satisfies $\theta_1 < \theta \leq \theta_2$, the control unit 16 determines bias of the sample solution has occurred on the end part 1a side of the test piece 1 in the lateral direction y. Although not particularly limited, the threshold value $\theta_2$ can be, for example, 45°. Then, the control unit 16 sets the analysis region to the analysis region R2 illustrated in Fig. 9 (Step S6).

In a case where the inclination $\theta$ satisfies $-\theta_2 \leq \theta < -\theta_1$, the control unit 16 determines bias of the sample solution has occurred on the end part 1b side of the test piece 1 in the lateral direction y. Then, the control unit 16 sets the analysis region to the analysis region R3 illustrated in Fig. 10 (Step S7).

Then, the control unit 16 creates concentration information of the analysis region set in Step S5, Step S6, or Step S7 (Step S8), performs analysis (the detection or quantification of antigens in the sample solution and the detection that the sample solution has appropriately flowed to the test piece 1) on the basis of the created concentration information, and notifies the notification unit 14 of the obtained analysis results (Step S9).

On the other hand, in a case where the inclination $\theta$ is $\theta < -\theta_2$ or $\theta > \theta_2$, the control unit 16 determines the bias of the sample solution has occurred and the bias is excessive, stops the analysis, and notifies the notification unit 14 of information showing an abnormality in posture of the test piece 1 (Step S10).

In this way, by changing the range, in the lateral direction y, of the analysis region set on the image on the basis of the inclination of the lateral axis Ay of the test piece 1 with respect to the horizontal, it is possible to suppress that the concentration value of the reaction section 4 (the determination line 5 and the control line 6) acquired from the image deviate from the original concentration value due to the bias of the sample solution, and the accuracy of the analysis based on the basis of the concentration value can be improved.

In addition, in the above-described example, a case where the range of the analysis region in the lateral direction y is changed in two stages of the analysis region R1 illustrated in Fig. 8 and the analysis region R2 illustrated in Fig. 9 or the analysis region R3 illustrating in Fig. 10 on the basis of the inclination $\theta$ of the lateral axis Ay of the test piece 1 in the relationship with one threshold value $\theta_1$ has been described. However, the ranges of analysis regions in the lateral direction y may be in multiple stages using a plurality of threshold values.

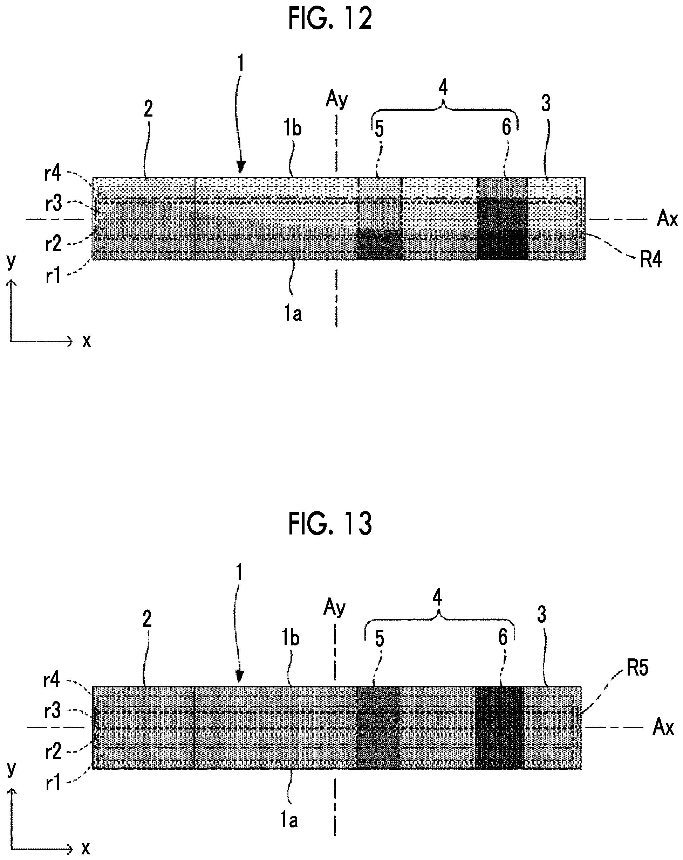

Fig. 12 illustrates another example of setting of the analysis region according to the inclination of the lateral axis Ay of the test piece 1 with respect to the horizontal.

The example of Fig. 12 shows an analysis region set in a case where the lateral axis Ay of the test piece 1 is inclined with respect to the horizontal, and the analysis region R4 is set to a range that covers the two divided region r2 and divided region r3 of which the central parts in the lateral direction y are adjacent to each other. The range of the analysis region R4 in the lateral direction y is not related with an inclination direction of the lateral axis Ay of the test piece 1, and is reduced toward a central part of the test piece 1 in the lateral direction y with respect to the analysis region R1 illustrated in Fig. 8.

In a case where the concentration information is created, the average value of the concentrations at the respective pixel positions within the analysis region R4 is calculated for every y-direction pixel row that extends in the lateral direction y of the test piece 1. However, also by virtue of the present example of setting, for example, a concentration value of a pixel group on an end part side having a relatively low coloration intensity is not included in the concentration value of the peak P1 corresponding to the determination line 5, and the difference between the concentration value of the peak P1 and the original concentration value becomes small. Accordingly, the accuracy of the analysis based on the concentration information can be improved.

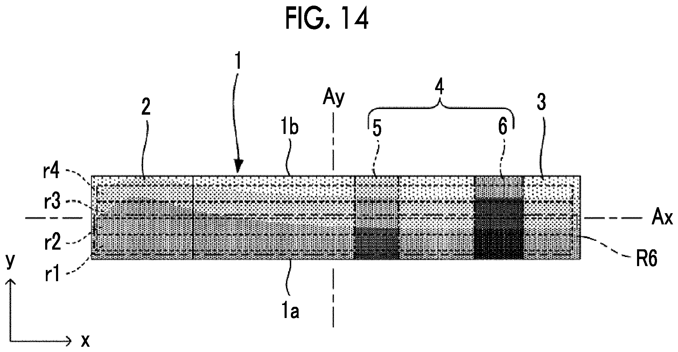

Figs. 13 and 14 illustrate other examples of setting of analysis regions according to the inclination of the lateral axis Ay of the test piece 1 with respect to the horizontal.

The example of Fig. 13 shows an analysis region set in a case where the lateral axis Ay of the test piece 1 is horizontal, and the example of Fig. 14 shows an analysis region set in a case where the lateral axis Ay of the test piece 1 is inclined with respect to the horizontal and the end part 1a out of the end part 1a and the end part 1b of the test piece 1 in the lateral direction y is disposed relatively below.

As illustrated in Fig. 13, in a case where the lateral axis Ay of the test piece 1 is horizontal, an analysis region R5 is set to a range that covers the two divided region r2 and divided region r3 of which central parts in the lateral direction y are adjacent to each other, out of the four divided region r1 to divided region r4.

Meanwhile, as illustrated in Fig. 14, in a case where the lateral axis Ay of the test piece 1 is inclined with respect to the horizontal and the end part 1a of the test piece 1 in the lateral direction y is disposed relatively below, an analysis region R6 is set to a range that covers two adjacent divided regions r1 and divided region r2 on the end part 1a side. That is, the range of the analysis region R6 in the lateral direction y is shifted toward the end part la side, which is disposed relatively below, out of the end part 1a and the end part 1b of the test piece 1 in the lateral direction y, in a state where the length in the lateral direction y is fixed with respect to the analysis region R5 illustrated in Fig. 13.

In addition, although illustration is omitted, in a case where the lateral axis Ay of the test piece 1 is inclined with respect to the horizontal and the end part 1b of the test piece 1 in the lateral direction y is disposed relatively below, an analysis region is set to a range that covers the two adjacent divided region r3 and divided region r4 on the end part 1b side, in other words, is shifted toward the end part lb, which is disposed relatively below, out of the end part la and the end part 1b of the test piece 1 in the lateral direction y, with respect to the analysis region R5 illustrated in Fig. 13.

In a case where the concentration information is created, the average value of the concentrations at the respective pixel positions within the analysis region R6 is calculated for every y-direction pixel row that extends in the lateral direction y of the test piece 1. However, also by virtue of the present example of setting, for example, a concentration value of a pixel group on an end part side having a relatively low coloration intensity is not included in the concentration value of the peak P1 corresponding to the determination line 5, and the difference between the concentration value of the peak P1 and the original concentration value becomes small. Accordingly, the accuracy of the analysis based on the concentration information can be improved.

Although a case where a timing at which the image of the test piece 1 is acquired is fixed to a timing after the predetermined reaction time $t_1$ has lapsed the start of the analysis has been described up to now, the acquisition timing of the image of the test piece 1 may be changed depending on the inclination of the longitudinal axis Ax of the test piece 1 with respect to the horizontal.

First, a flow of the sample solution in a case where the longitudinal axis Ax of the test piece 1 is horizontal is used as a basis. In a case where the longitudinal axis Ax of the test piece 1 is inclined with respect to the horizontal and the downstream side of the flow of the sample solution is disposed relatively above, the flow of the sample solution becomes slower due to the influence of gravity. On the other hand, in a case where the downstream side of the flow of the sample solution is disposed relatively below, the flow of the sample solution becomes faster due to the influence of gravity.

The above predetermined reaction time $t_1$ is the time during which the reaction section 4 (the determination line 5 and the control line 6) is colored with sufficient intensity for detection in a case where the sample solution including antigens in a predetermined concentration has flowed through the horizontally-placed test piece 1. In a case where the flow of the sample solution becomes slower, the amount of the sample solution passing through the reaction section 4 within the predetermined reaction time $t_1$ becomes smaller, and the coloration of the reaction section 4 becomes weaker. On the other hand, in a case where the flow of the sample solution becomes faster, the amount of the sample solution passing through the reaction section 4 becomes less within the predetermined reaction time $t_1$, and the coloration of the reaction section 4 becomes stronger.

In a case where the acquisition timing of the image of the test piece 1 is fixed to the predetermined reaction time $t_1$, there is a concern that the concentration value of the peak P1 corresponding to the determination line 5 and the concentration value of the peak P2 corresponding to a control line 6 in the concentration information created from the acquired image may fluctuate due to the inclination of the longitudinal axis Ax of the test piece 1 with respect to the horizontal even the same the sample solution, and there is concern that the accuracy of the analysis based on the concentration value of the peak P1 and the concentration value of the peak P2 may decrease. Thus, in a method of creating the concentration information to be described below, the acquisition timing of the image of the test piece 1 is changed on the basis of the inclination of the longitudinal axis Ax of the test piece 1 with respect to the horizontal.

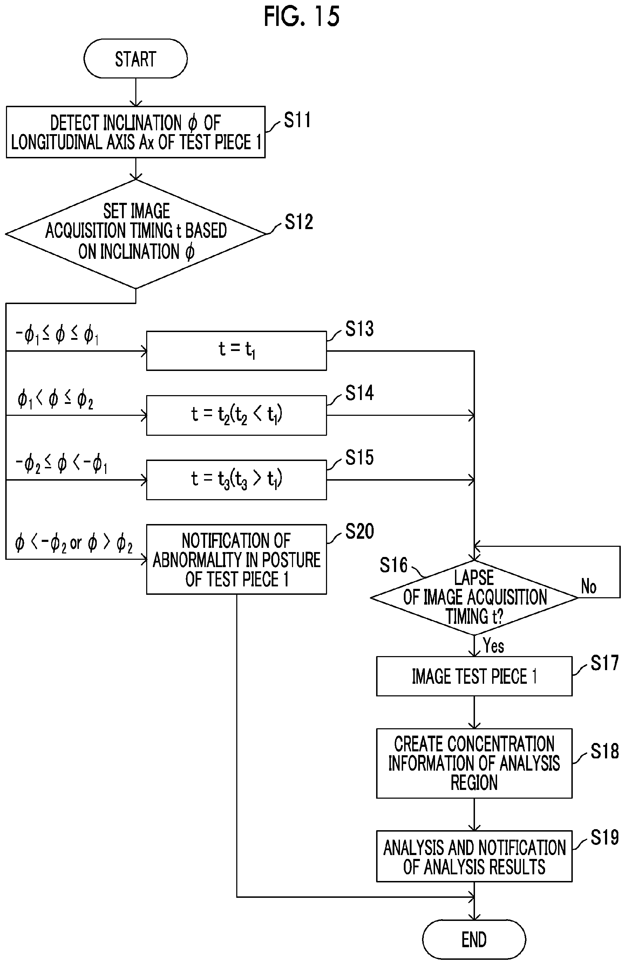

Fig. 15 illustrates a flow of the processing, in which the acquisition timing of the image is changed in accordance with the inclination of the longitudinal axis Ax of the test piece 1 with respect to the horizontal, to be executed the control unit 16.

In a case where an analysis start instruction is input, the control unit 16 acquires the inclination of the installation unit 20 of the imaging unit 12 with respect to the horizontal from the inclination detecting unit 13, and detects an inclination $\phi$ of the longitudinal axis Ax of the test piece 1 installed in the installation unit 20 with respect to the horizontal (Step S11).

The control unit 16 sets the image acquisition timing t is set on the basis of the inclination $\phi$ of the longitudinal axis Ax of the test piece 1 detected in Step S11 (Step S12). In the present example, the control unit 16 applies two threshold value $\phi_1$ and threshold value $\phi_2$ to the inclination $\phi$, and sets the image acquisition timing t. In addition, both the threshold value $\phi_1$ and the threshold value $\phi_2$ are positive values, and satisfy $\phi_1 < \phi_2$. Additionally, an inclination direction in which the downstream side of the flow of the sample solution is disposed relatively below is set as the normal direction.

In a case where the inclination $\phi$ satisfies $-\phi_1 \leq \phi \leq \phi_1$, the control unit 16 determines that the flow rate of the sample solution can be regarded as being the same as the flow rate in a case where the sample solution flows through the horizontal test piece 1. Although not particularly limited, the threshold value $\phi_1$ can be, for example, 10°. Then, the control unit 16 sets the image acquisition timing t to the above predetermined reaction time $t_1$ (Step S13).

In a case where the inclination $\phi$ satisfies $\phi_1 < \phi \leq \phi_2$, the control unit 16 determines that the flow rate of the sample solution is faster than the flow rate in a case where the sample solution flows through the horizontal test piece 1. Although not particularly limited, the

threshold value $\phi_2$ can be, for example, 45°. Then, the control unit 16 sets the image acquisition timing t to a time $t_2$ shorter than the above predetermined reaction time $t_1$ (Step S14).

In a case where the inclination $\phi$ satisfies $-\phi_2 \leq \phi < -\phi_1$, the control unit 16 determines that the flow rate of the sample solution is slower than the flow rate in a case where the sample solution flows through the horizontal test piece 1. Then, the control unit 16 sets the image acquisition timing t to a time $t_3$ longer than the above predetermined reaction time $t_1$ (Step S15).

Then, the control unit 16 counts the lapse of the image acquisition timing t set in Step S13, Step S14, or Step S15 (Step S16).

The control unit 16 makes the imaging unit 12 image the test piece 1 after the lapse of the image acquisition timing t (Step S17). An image of the test piece 1 acquired by the imaging unit 12 is input to the control unit 16 (image processing unit 23).

Then, the control unit 16 creates concentration information of the analysis region of the image (Step S18), performs analysis (the detection or quantification of antigens in the sample solution and the detection that the sample solution has appropriately flowed to the test piece 1) on the basis of the created concentration information, and notifies the notification unit 14 of the obtained analysis results (Step S19).

In addition, the analysis region is appropriately set to any one example of setting out of the examples of setting illustrated in Figs. 8 to 10, the example of setting illustrated in Fig. 12, or the examples of setting illustrated in Figs. 13 and 14, depending on the inclination of the lateral axis Ay of the test piece 1 with respect to the horizontal, as described above.

On the other hand, in a case where the inclination $\phi$ satisfies $\phi < -\phi_2$ or $\phi > \phi_2$, the control unit 16 determines that the flow of the sample solution becomes excessively slow or excessively fast, stops the analysis, and notifies the notification unit 14 of information showing an abnormality in posture of the test piece 1 (Step S20).

In this way, by changing the image acquisition timing on the basis of the inclination of the longitudinal axis Ax of the test piece 1 with respect to the horizontal, the amount of the sample solution passing through the reaction section 4 until the image acquisition timing can be stabilized irrespective of the flow rate of the sample solution. Accordingly, the concentration value of the reaction section 4 (the determination line 5 and the control line 6) acquired from the image with respect to the same the sample solution can be stabilized, and the accuracy of the analysis based on the concentration value can be further improved.

In addition, in the above-described example, a case where the image acquisition timing is changed in the two stages of t1 and t2 in a case where the image acquisition timing is advanced on the basis of the inclination $\phi$ of the longitudinal axis Ax of the test piece 1 in a relationship with one threshold value $\phi_1$ and the image

acquisition timing is changed in the two stages of t1 and t3 in a case where the image acquisition timing was delayed has been described. However, the image acquisition timing may be changed in multiple stages using a plurality of threshold values.

As described above, an immunological examination apparatus disclosed in the present specification comprises an imaging unit that acquires an image of a chromatographic test piece in which a sample solution is made to flow in a longitudinal direction; an inclination detecting unit that detects an inclination of a lateral axis of the chromatographic test piece with respect to the horizontal; and an analysis unit that sets a single analysis region to the image acquired by the imaging unit, changes a range of the analysis region in a lateral direction on the basis of inclination of the lateral axis detected by the inclination detecting unit, and analyzes the sample solution on the basis of concentration information of the changed analysis region.

Additionally, with respect to the analysis region in a case where the lateral axis is horizontal, the analysis unit reduces the analysis region toward an end part, which is disposed relatively below, out of both end parts of the chromatographic test piece in the lateral direction in a case where the lateral axis is inclined.

Additionally, with respect to the analysis region in a case where the lateral axis is horizontal, the analysis unit reduces the analysis region toward a central part of the chromatographic test piece in the lateral direction in a case where the lateral axis is inclined.

Additionally, with respect to the analysis region in a case where the lateral axis is horizontal, the analysis unit shifts the analysis region in the lateral direction toward an end part, which is disposed relatively below, out of both end parts of the chromatographic test piece in the lateral direction in a case where the lateral axis is inclined.

The analysis unit stops an analysis in a case where the inclination of the lateral axis exceeds a predetermined angle, and the immunological examination apparatus further comprises a notification unit that gives a notification of the stop of the analysis.

Additionally, the inclination detecting unit further detects an inclination of a longitudinal axis of the chromatographic test piece with respect to the horizontal, and with respect to an acquisition timing of the image in a case where the longitudinal axis is horizontal, the imaging unit delays the acquisition timing of the image in a case where a downstream side of a flow of the sample solution that flows through the chromatographic test piece in the longitudinal direction is disposed relatively above, and advances the acquisition timing of the image in a case where the downstream side of the flow of the sample solution is disposed relatively below.

Additionally, the analysis unit stops an analysis in a case where the inclination of the longitudinal axis exceeds a predetermined angle, and the immunological examination apparatus further comprises a notification unit that gives a notification of the stop of the analysis.

The invention can improve analysis accuracy irrespective of the posture of the chromatographic test piece.

Although the embodiment of the invention has been described in detail above, this is merely an example, and the invention can be carried out in aspects to which various changes are added, without departing from the scope of the appended claims. The present application is based on Japanese Patent application (JP2017-021611) filed on February 8, 2017.

Explanation of References

1: chromatographic test piece
la: end part in lateral direction
1b: end part in lateral direction
2: dropping section
3: spreading section
4: reaction section
5: determination line
6: control line
7: cartridge
8: opening
10: immunological examination apparatus
11: operating unit
12: imaging unit
13: inclination detecting unit
14: notification unit
15: storage unit
16: control unit
20: installation unit
20a: upper surface of installation unit
21: light source
22: imaging element
23: image processing unit
24: analysis unit
a: antigen
b: labeled antibody
ab: antigen-antibody complex
c: first capture antibody
d: second capture antibody
Ax: longitudinal axis
Ay: lateral axis
P1: peak
P2: peak
r1: divided region
r2: divided region
r3: divided region
r4: divided region
R1: analysis region
R2: analysis region
R3: analysis region
R4: analysis region
R5: analysis region
R6: analysis region

t: image acquisition timing
t1: reaction time
t2: time
t3: time
x: longitudinal direction
y: lateral direction
$\theta_1$: threshold value
$\theta_2$: threshold value
$\phi_1$: threshold value
$\theta_2$: threshold value

**Claims**

1.  An immunological examination apparatus (10) comprising:

    an imaging unit (12) adapted to acquire an image of a chromatographic test piece (1) on which a sample solution is made to flow in a longitudinal direction (X);
    an inclination detecting unit (13) adapted to detect an inclination of a lateral axis (Ay) of the chromatographic test piece (1) with respect to the horizontal; and
    an analysis unit (24) adapted to set a single analysis region (R1, R2...) to the image acquired by the imaging unit (12) to change a range of the analysis region in a lateral direction (Y) on the basis of the inclination of the lateral axis (Ay) detected by the inclination detecting unit (13), and to analyze the sample solution on the basis of concentration information, i.e. brightness and/or luminance, of the analysis region that is changed.

2.  The immunological examination apparatus (10) according to claim 1,
    wherein, in a case where the lateral axis is inclined, the analysis unit (24) is adapted to reduce the analysis region (R1, R2...) toward an end part which is disposed relatively below out of end parts of the chromatographic test piece (1) in the lateral direction relative to the analysis region in a case where the lateral axis is horizontal,.

3.  The immunological examination apparatus (10) according to claim 1,
    wherein, in a case where the lateral axis is inclined, the analysis unit (24) is adapted to reduce the analysis region toward a central part of the chromatographic test piece in the lateral direction relative to the analysis region in a case where the lateral axis is horizontal,.

4.  The immunological examination apparatus (10) according to claim 1,
    wherein, in a case where the lateral axis is inclined, the analysis unit (24) is adapted to shift the analysis region in the lateral direction toward an end part which is disposed relatively below out of end parts of the chromatographic test piece (1) in the lateral direction relative to the analysis region in a case where the lateral axis is horizontal.

5. The immunological examination apparatus (10) according to any one of claims 1 to 4, wherein the analysis unit (24) is adapted to stop an analysis in a case where the inclination of the lateral axis exceeds a predetermined angle, and the immunological examination apparatus (10) further comprises a notification unit (14) adapted to give a notification of a stop of the analysis.

6. The immunological examination apparatus (10) according to any one of claims 1 to 5, wherein the inclination detecting unit (13) is further adapted to detect an inclination of a longitudinal axis of the chromatographic test piece (1) with respect to the horizontal, and wherein, relative to an acquisition timing of the image in a case where the longitudinal axis is horizontal, the imaging unit (12) is adapted to delay the acquisition timing of the image in a case where a downstream side of a flow of the sample solution that flows on the chromatographic test piece (1) in the longitudinal direction is disposed relatively above, and to advance the acquisition timing of the image in a case where the downstream side of the flow of the sample solution is disposed relatively below.

7. The immunological examination apparatus (10) according to claim 6, wherein the analysis unit (24) is adapted to stop an analysis in a case where the inclination of the longitudinal axis exceeds a predetermined angle, and the immunological examination apparatus (10) further comprises a notification unit (14) is adapted to give a notification of a stop of the analysis.

**Patentansprüche**

1. Immunologische Testvorrichtung (10), umfassend:

   eine Bildgebungseinheit (12), ausgebildet zum Erfassen eines Bilds eines chromatographischen Prüfteils (1), an welchem eine Probenlösung zum Strömen in einer Längsrichtung (X) gebracht wird; eine Neigungsdetektoreinheit (13), ausgebildet zum Nachweisen einer Neigung einer seitlichen Achse (Ay) des chromatographischen Prüfteils (1) in Bezug auf die Horizontale; und eine Analyseeinheit (24), ausgebildet zum Einstellen einer einzelnen Analysezone (R1, R2, ...) an dem von der Bildgebungseinheit (12) erfassten Bild, um einen Bereich der Analysezone in einer Seitenrichtung (Y) auf der Grundlage der Neigung der Seitenachse (Ay) zu ändern, die von der Neigungsdetektoreinheit (13) nachgewiesen wurde, und zum Analysieren der Probenlösung auf der Grundlage von Konzentrationsinformation, das heißt der Helligkeit und/oder Luminanz der geänderten Analysezone.

2. Immunologische Prüfvorrichtung (10) nach Anspruch 1, bei der für den Fall, dass die Seitenachse geneigt ist, die Analyseeinheit (24) ausgebildet ist zum Reduzieren der Analysezone (R1, R2, ...) in Richtung eines Endteils, der sich relativ unterhalb von Endteilen des chromatographischen Prüfteils (1) in der Seitenrichtung relativ zu der Analysezone für den Fall befindet, dass die Seitenachse horizontal verläuft.

3. Immunologische Prüfvorrichtung (10) nach Anspruch 1, bei der für den Fall, dass die Seitenachse geneigt ist, die Analyseeinheit (24) ausgebildet ist zum Reduzieren der Analysezone in Richtung eines Mittelteils des chromatographischen Prüfteils in Seitenrichtung relativ zu der Analysezone für den Fall, dass die Seitenachse horizontal verläuft.

4. Immunologische Prüfvorrichtung (10) nach Anspruch 1, bei der für den Fall, dass die Seitenachse geneigt ist, die Analyseeinheit (24) ausgebildet ist zum Verschieben der Analysezone in der seitlichen Richtung zu einem Endteil hin, das sich relativ unterhalb von Endteilen des chromatographischen Prüfteils (1) in der Seitenrichtung befindet, bezogen auf die Analysezone für den Fall, dass die Seitenachse horizontal verläuft.

5. Immunologische Prüfvorrichtung (10) nach einem der Ansprüche 1 bis 4, bei der die Analyseeinheit (24) ausgebildet ist zum Anhalten einer Analyse für den Fall, dass die Neigung der Seitenachse einen vorbestimmten Winkel übersteigt, und die immunologische Prüfvorrichtung (10) weiterhin eine Meldeeinheit (14) aufweist, ausgebildet zum Ausgeben einer Meldung über das Anhalten der Analyse.

6. Immunologische Prüfvorrichtung (10) nach einem der Ansprüche 1 bis 5, bei der die Neigungsdetektoreinheit (13) weiterhin ausgebildet ist zum Nachweisen einer Neigung einer Längsachse des chromatographischen Prüfteils (1) in Bezug auf die Horizontale, und

wobei relativ zu einem Erfassungszeitpunkt des Bilds für den Fall, dass die Längsachse horizontal verläuft, die Bildgebungseinheit (12) ausgebildet ist zum Verzögern des Erfassungszeitpunkts des Bilds für den Fall, dass eine stromabwärtige Seite einer Strömung der Probenlösung, die auf dem chromatographischen Prüfteil (1) in der Längsrichtung strömt, relativ oberhalb angeordnet ist, und zum Vorrücken des Erfassungszeitpunkts des Bilds für den Fall, dass die stromabwärtige Seite der Strömung der Probenlösung sich relativ unten befindet.

7. Immunologische Prüfvorrichtung (10) nach Anspruch 6,
   bei der die Analyseeinheit (24) ausgebildet ist zum Anhalten einer Analyse für den Fall, dass die Neigung der Längsachse einen vorbestimmten Winkel übersteigt, und
   die immunologische Prüfvorrichtung (10) weiterhin eine Meldeeinheit (14) aufweist, ausgebildet zum Ausgeben einer Meldung bezüglich eines Anhaltens der Analyse.

## Revendications

1. Appareil d'examen immunologique (10), comprenant :

   une unité d'imagerie (12) apte à acquérir une image d'une éprouvette chromatographique (1) sur laquelle il est fait en sorte qu'une solution d'échantillon s'écoule dans une direction longitudinale (X) ;
   une unité de détection d'inclinaison (13), apte à détecter une inclinaison d'un axe latéral (Ay) de l'éprouvette chromatographique (1) par rapport à l'horizontale, et
   une unité d'analyse (24) apte à régler une région d'analyse unique (R1, R2 ...) sur l'image acquise par l'unité d'imagerie (12) afin de modifier une plage de la région d'analyse dans une direction latérale (Y) sur la base de l'inclinaison de l'axe latéral (Ay) détectée par l'unité de détection d'inclinaison (13), et analyser la solution d'échantillon sur la base des informations de concentration, à savoir la luminosité et/ou la luminance, de la région d'analyse qui est modifiée.

2. Appareil d'examen immunologique (10) selon la revendication 1,
   dans lequel dans un cas où l'axe latéral est incliné, l'unité d'analyse (24) est apte à réduire la région d'analyse (R1, R2 ...) vers une partie d'extrémité, laquelle est disposée relativement au-dessous parmi des parties d'extrémité de l'éprouvette chromatographique (1) dans la direction latérale par rapport à la région d'analyse dans un cas où l'axe latéral est horizontal.

3. Appareil d'examen immunologique (10) selon la revendication 1,
   dans lequel dans un cas où l'axe latéral est incliné, l'unité d'analyse (24) est apte à réduire la région d'analyse vers une partie centrale de l'éprouvette chromatographique dans la direction latérale par rapport à la région d'analyse dans un cas où l'axe latéral est horizontal.

4. Appareil d'examen immunologique (10) selon la revendication 1,
   dans lequel dans un cas où l'axe latéral est incliné, l'unité d'analyse (24) est apte à décaler la région d'analyse dans la direction latérale vers une partie d'extrémité, laquelle est disposée relativement vers le bas parmi les parties d'extrémité de l'éprouvette chromatographique (1) dans la direction latérale par rapport à la région d'analyse dans un cas où l'axe latéral est horizontal.

5. Appareil d'examen immunologique (10) selon l'une quelconque des revendications 1 à 4,
   dans lequel l'unité d'analyse (24) est apte à arrêter une analyse dans un cas où l'inclinaison de l'axe latéral dépasse un angle prédéterminé, et
   l'appareil d'examen immunologique (10) comprend en outre une unité de notification (14) apte à donner une notification d'un arrêt de l'analyse.

6. Appareil d'examen immunologique (10) selon l'une quelconque des revendications 1 à 5,
   dans lequel l'unité de détection d'inclinaison (13) est en outre apte à détecter une inclinaison d'un axe longitudinal de l'éprouvette chromatographique (1) par rapport à l'horizontale, et
   dans lequel par rapport à une temporisation d'acquisition de l'image dans un cas où l'axe longitudinal est horizontal, l'unité d'imagerie (12) est apte à retarder la temporisation d'acquisition de l'image dans un cas où un côté aval d'un flux de la solution d'échantillon, laquelle s'écoule sur l'éprouvette chromatographique (1) dans la direction longitudinale, est disposé relativement au-dessus, et à avancer la temporisation d'acquisition de l'image dans un cas où le côté aval du flux de la solution d'échantillon est disposé relativement au-dessous.

7. Appareil d'examen immunologique (10) selon la revendication 6,
   dans lequel l'unité d'analyse (24) est apte à arrêter une analyse dans un cas où l'inclinaison de l'axe longitudinal dépasse un angle prédéterminé, et
   l'appareil d'examen immunologique (10) comprend en outre une unité de notification (14) apte à donner une notification d'un arrêt de l'analyse.

## FIG. 1

FIG. 2

# FIG. 3

10

```
ANALYSIS APPARATUS

11 ── OPERATING
       UNIT

                        CONTROL UNIT                INCLINATION
                                                    DETECTING UNIT ── 13
                         IMAGE
                      PROCESSING UNIT
                                        23

12 ── IMAGING UNIT                                  NOTIFICATION
                                                    UNIT           ── 14
21 ── LIGHT             ANALYSIS UNIT
       SOURCE
                                        24
22 ── IMAGING                                       STORAGE UNIT   ── 15
       ELEMENT

                            16
```

# FIG. 4

# FIG. 5

FIG. 6

FIG. 7

## FIG. 8

## FIG. 9

FIG. 10

## FIG. 11

START

S1 — LAPSE OF REACTION TIME $t_i$? — No

Yes

IMAGE TEST PIECE 1 — S2

DETECT INCLINATION $\theta$ OF LATERAL AXIS Ay OF TEST PIECE 1 — S3

SET ANALYSIS REGION BASED ON INCLINATION $\theta$ — S4

$-\theta_1 \leq \theta \leq \theta_1$ — SET ANALYSIS REGION TO R1 — S5

$\theta_1 < \theta \leq \theta_2$ — SET ANALYSIS REGION TO R2 — S6

$-\theta_2 \leq \theta < -\theta_1$ — SET ANALYSIS REGION TO R3 — S7

$\theta < -\theta_2$ or $\theta > \theta_2$ — NOTIFICATION OF ABNORMALITY IN POSTURE OF TEST PIECE 1 — S10

CREATE CONCENTRATION INFORMATION OF ANALYSIS REGION — S8

ANALYSIS AND NOTIFICATION OF ANALYSIS RESULTS — S9

END

21

## FIG. 12

## FIG. 13

FIG. 14

## FIG. 15

START

S11 — DETECT INCLINATION $\phi$ OF LONGITUDINAL AXIS Ax OF TEST PIECE 1

S12 — SET IMAGE ACQUISITION TIMING t BASED ON INCLINATION $\phi$

$-\phi_1 \leq \phi \leq \phi_1$

S13 — $t = t_1$

$\phi_1 < \phi \leq \phi_2$

S14 — $t = t_2 (t_2 < t_1)$

$-\phi_2 \leq \phi < -\phi_1$

S15 — $t = t_3 (t_3 > t_1)$

$\phi < -\phi_2$ or $\phi > \phi_2$

S20 — NOTIFICATION OF ABNORMALITY IN POSTURE OF TEST PIECE 1

S16 — LAPSE OF IMAGE ACQUISITION TIMING t? — No

Yes

S17 — IMAGE TEST PIECE 1

S18 — CREATE CONCENTRATION INFORMATION OF ANALYSIS REGION

S19 — ANALYSIS AND NOTIFICATION OF ANALYSIS RESULTS

END

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009115521 A
- US 20090253213 A
- EP 2320232 A
- JP 2014035290 A
- CN 105353115
- EP 2703803 A
- WO 2013014540 A
- EP 1111386 A
- JP 2017021611 A